Europäisches Patentamt

(19) European Patent Office

Office européen des brevets

(11) **EP 1 486 204 A1**

(12) **EUROPEAN PATENT APPLICATION**
published in accordance with Art. 158(3) EPC

(43) Date of publication:
**15.12.2004 Bulletin 2004/51**

(21) Application number: **03708640.2**

(22) Date of filing: **17.03.2003**

(51) Int Cl.[7]: **A61K 9/72**, A61K 9/51,
A61K 47/14, A61K 47/24,
A61K 47/28, A61K 47/32

(86) International application number:
**PCT/JP2003/003165**

(87) International publication number:
**WO 2003/077891 (25.09.2003 Gazette 2003/39)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **18.03.2002 US 365752 P
18.03.2002 US 365697 P**

(71) Applicant: **YAMANOUCHI PHARMACEUTICAL
CO. LTD.
Tokyo 103-8411 (JP)**

(72) Inventors:
• **Katsuma, Masataka**
**Yaizu-shi, Shizuoka 425-0072 (JP)**
• **Kawai, Hitoshi**
**Yaizu-shi, Shizuoka 425-0072 (JP)**
• **Mizumoto, Takao**
**Yaizu-shi, Shizuoka 425-0072 (JP)**

(74) Representative: **Bates, Philip Ian
Reddie & Grose
16 Theobalds Road
London WC1X 8PL (GB)**

(54) **POWDERY MEDICINAL COMPOSITIONS FOR INHALATION AND PROCESS FOR
PRODUCING THE SAME**

(57)    The present invention provides marked results in that it is possible to present by a simple method a dry powder inhalation for pulmonary delivery that is made from a biologically active substance in crystal form and a biocompatible, electrostatic aggregation-inhibiting substance and that has excellent safety, stability, and pulmonary delivery performance.

Moreover, it is also possible to provide sustained release performance that is appropriate for the properties of the biologically active substance by selecting [the appropriate] hydrophobic substance.

**EP 1 486 204 A1**

**Description**

**Technical Field**

The present invention relates to a dry powder inhalation for pulmonary delivery with improved pulmonary delivery performance and a manufacturing method thereof. In particular, the present invention pertains to a dry powder inhalation for pulmonary delivery showing excellent pulmonary delivery performance which is obtained by coating a fine biologically active substance in crystal form with a specific electrostatic aggregation-inhibiting substance, and a manufacturing method thereof.

**Background of the Invention**

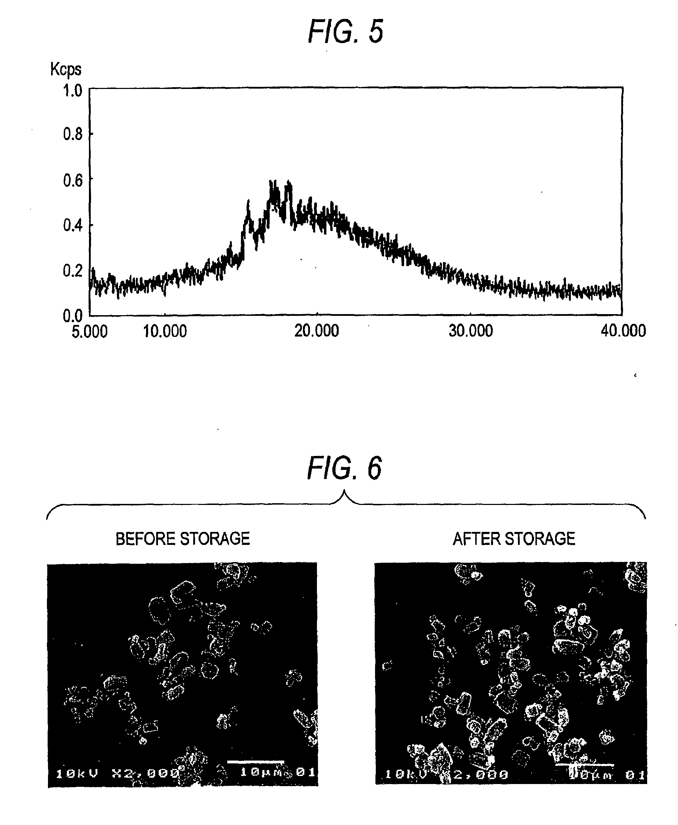

Inhalation administration has been widely used in the past as a therapeutic administration route for local disease in the lung, such as asthma, because it is possible to deliver directly to the lungs biologically active substance particles that have been made very fine. The powder particles that are handled in the field of inhalations are for the purpose of pulmonary (bronchial, bronchiolar, alveolar) delivery and therefore, are extremely fine (10 μm or smaller) when compared to powders and pharmaceutical preparations that are generally handled in the field of oral agents (several ten μm to several hundred μm). Nevertheless, such fine powder particles induce adhesion and aggregation between particles and dispersibility in the gas phase deteriorates. Therefore, there is concern that there will not be sufficient pulmonary delivery: Moreover, even if good pulmonary delivery performance is obtained, it is desirable in terms of quality assurance that adhesion and aggregation do not occur over time during storage.
In recent years there has been an abundance of studies of pulmonary delivery formulations with inhalations of peptides and proteins the purpose of which is systemic absorption, that is, absorption into the blood. Nevertheless, although absorptivity is excellent with inhalation administration, these powder particles are extremely fine and thus, the biologically active substance dissolves quickly and the excellent absorption by the pulmonary mucosa that follows results in the time for which the drug effects of the biologically active substance act being short. There are therefore cases in which frequent administration is inevitable. Furthermore, there are concerns over the occurrence of systemic adverse effects in the case of biologically active substances that display extremely strong effects because of this excellent absorptivity.
Consequently, there is a demand in the development of powder inhalations for pharmaceutical preparations that not only improve pulmonary delivery performance of dry powder inhalations for pulmonary delivery containing a biologically active substance, but also are very safe, have excellent stability over time, and, depending on the case, can be given sustained-release performance in order to prolong the time for which they are effective in accordance with the biologically active substance that is used.
There have been various attempts in the past aimed at improving pulmonary delivery performance. Mokhtar et al. prepared sustained-release microspheres for inhalation using polylactic acid, which is a base with *in vivo* degradability, and report on their pulmonary delivery performance, and the like ([non-patent reference 1] Int. J. Pharm. 175, 135-145 (1998)). Moreover, an invention is reported relating to a particle system for pulmonary delivery containing biodegradable particles with tap density, which is an indicator of bulk density when packed by tapping, of less than 0.4 g/cm$^3$, wherein mass-average diameter is between 5μm and 30 μm ([patent reference 1] International Publication Pamphlet No. WO98/31346 (corresponds to Japanese Patent Application, National Publication 2001-526634)). In particular, it is disclosed that particle aggregation is avoided and pulmonary delivery performance is improved as a result of making sufficiently light particles by designing particles having a specific tap density and a specific average diameter in order to produce particles having a specific aerodynamic diameter. In addition, it also contends that release of biologically active substance inside the lungs can be controlled and that sustained release performance is enhanced by adding cholesterol.
Nevertheless, although these microspheres for inhalation show good pulmonary delivery performance, a process whereby the biologically active substance is dissolved is used and therefore, there is concern that the biologically active substance will not retain its stability, leading to aggregation and the like, of microparticles over time and a reduction in pulmonary delivery performance. Furthermore, according to the latter Specification, particularly the results of *in vitro* dissolution tests shown in Figure 6, the composition of this technology shows the release all at once of 40% or more of the biologically active substance in the early stages after starting the test and therefore, there is also room for improvement in terms of providing sustained release performance because sufficiently controlled dissolution is not realized.
A microparticle composition for pulmonary delivery which contains biologically active substance and biodegradable substance, is made from biocompatible particles, and has the properties of a tap density of less than 0.4g/cm$^3$, a geometric particle diameter of 5 to 30 μm, and an aerodynamic particle diameter of 1 to 5 μm is disclosed as another technology ([patent reference 2] International Publication Pamphlet WO 97/44103 (corresponds to Japanese Patent National Publication 2000-511189)). As with the above-mentioned invention, there is a need for improved stability with this invention as well because a process whereby the biologically active substance is dissolved is used.

Moreover, an invention relating to the use of a surfactant in the production of a drug for pulmonary delivery is disclosed. This drug comprises multiple porous fine structures that have been made into an aerosol using an inhalation device for presentation of an aerosol drug comprising a biologically active agent, and this aerosol drug is an administration form that is [administered] to the nose or part of the airway of the lungs of a patient requiring at least this aerosol drug ([patent reference 3] International Publication Pamphlet WO 99/16419 (corresponds to Japanese Patent National Publication 2001-517691). It is disclosed that by means of this invention it is possible to present a powder that has stable dispersibility, reduces attraction between particles, and shows relatively low cohesive force suitable for use of powder inhalations with a hollow or porous fine structure with the standard particle diameter and low bulk density. Nevertheless, special manufacturing methods and manufacturing conditions, including the addition of volatile substances, are necessary in order to realize such a porous fine structure as this aerosol drug. In addition, there is the concern that this drug will be amorphous because of the manufacturing method whereby the drug is dissolved and there are concerns over not only [problems] with stability of the drug itself during storage under harsh conditions, but also aggregation between particles.

As previously mentioned, various attempts have been made to improve pulmonary delivery performance of powder particles in the field of powder inhalations, but there is still room for improvement of dry powder inhalations for pulmonary delivery that can be prepared by a simple method and are very safe, have excellent stability over time, show excellent pulmonary delivery performance, and depending on the case, can be given sustained-release performance in accordance with the biologically active substance that is used.

**Disclosure of the Invention**

In light of these conditions, the inventors performed intense research in order to overcome the above-mentioned problems and as a result, they discovered that when a specific electrostatic aggregation-inhibiting substance having a phase transition temperature and/or melting point of 40°C or higher, such as cholesterol or hydrogenated lecithin, is selected and a fine biologically active substance powder in crystal form is coated with the above-mentioned specific substance, cohesiveness of the particles themselves is improved over powder particles containing a crystalline biologically active substances that has not been coated, or that have been coated with the above-mentioned specific substance but contain a biologically active substance that is not in crystal form, and therefore, good pulmonary delivery performance is obtained and excellent pulmonary delivery performance is realized even after storage over time under high-temperature conditions. Furthermore, the present invention was successfully completed upon discovering that when, of these specific substances, a hydrophobic substance is used, the coated particles show sustained dissolution whereby by means of the dissolution test method discussed later, dissolution during the early stages of the test is sufficiently controlled.

Although the mechanism by which pulmonary delivery performance is markedly improved by the present invention is not yet clear, it is estimated that by coating the biologically active substance in crystal form with the above-mentioned specific substance, biologically active substance is not exposed where the powder particles contact one another and adhesion between powder particles is reduced. Moreover, with regard to sustained release performance, it is estimated that because there is less biologically active substance that contacts the dissolution test fluid when compared to technology with which amorphous biologically active substance is exposed (matrix technology that includes an amorphous biologically active substance), a more hydrophobic environment is made and penetration by dissolution test fluid is delayed, and the like, resulting in the realization of sustained release. It is estimated that dissolution of biologically active substance is controlled during the early stages when dissolution tests are started because biologically active substance in amorphous form is not exposed.

Dissolution usually is fast due to an increase in surface area when the particles are fine and it was a completely unexpected finding that sustained release can be realized, and stability and pulmonary delivery performance can also be realized even if a biologically active substance in crystal form is used.

Incidentally, a powder inhalation obtained by making a drug into fine [particles] in a state of being suspended in an aqueous polymer solution together with lubricant and then spray drying is disclosed ([patent reference 3]. Japanese Kokai Patent No. 11-79985). By means of the technology in question, lubricant is adhered to the drug with polymer so that lubricant is scattered over the surface of the drug and as a result, there is a reduction in the amount that deposits on the powder dispersing device and dispersibility in the gas phase is improved. The amount of this lubricant is 1 to 3 wt% and therefore, this is different from the technical means of the present invention, the purpose of which is to coat the biologically active substance. Moreover, it is essential to use a polymer substance as the adhesion base in the technology in question. The hydroxypropylcellulose, and the like, that is used does not decompose *in vivo* and therefore, there is also concern over accumulation in the lungs.

That is, the present invention pertains to

1. a dry powder inhalation for pulmonary delivery, characterized in that is obtained by coating a biologically active substance in crystal form having a particle diameter of 0.5 μm to 8 μm with a biocompatible, electrostatic aggregation-inhibiting substance having a melting point and/or phase transition temperature of 40°C or higher, and it has a particle diameter of 0.5 to 8 μm,

2. a dry powder inhalation for pulmonary delivery according to above-mentioned 1, wherein the biocompatible, electrostatic aggregation-inhibiting substance having a melting point and/or phase transition temperature of 40°C or higher is one or two or more selected from the group consisting of hydrogenated lecithin, distearoyl phosphatidylcholine, cholesterol, cholesterol palmitate, cholesterol stearate, polyoxyethylene-polyoxypropylene glycol, polyethylene glycol 4000, polyethylene glycol 6000, polyethylene glycol 20000, and L-cystine,

3. a dry powder inhalation for pulmonary delivery according to above-mentioned 2, wherein the biocompatible, electrostatic aggregation-inhibiting substance having a melting point and/or phase transition temperature of 40°C or higher is one or two or more selected from the group consisting of hydrogenated lecithin, cholesterol, distearoyl-phosphatidylcholine, and polyethylene glycol 4000,

4. a dry powder inhalation for pulmonary delivery according to above-mentioned 3, wherein the biocompatible, electrostatic aggregation-inhibiting substance having a melting point and/or phase transition temperature of 40°C or higher is one or two or more selected from the group consisting of hydrogenated lecithin and cholesterol,

5. a dry powder inhalation for pulmonary delivery according to above-mentioned 2, characterized in that it contains 0.05 to 95 wt% biologically active substance and 5 to 99.95 wt% biocompatible, electrostatic aggregation-inhibiting substance having a melting point and/or phase transition temperature of 40°C or higher, it is obtained by coating the biologically active substance with this [biocompatible]substance, and it has a geometric particle diameter of 0.5 to 8 μm,

6. a method of manufacturing a dry powder inhalation for pulmonary delivery, characterized in that [the powder] contains a biologically active substance in crystal form having a particle diameter of 0.5 μm to 8 μm and/or a biocompatible, electrostatic aggregation-inhibiting substance having a melting point and/or phase transition temperature of 40°C or higher, it is obtained by coating the above-mentioned biologically active substance with this [biocompatible] substance, and it has a geometric particle diameter of 0.5 to 8μm,

7. a method of manufacturing a dry powder inhalation for pulmonary delivery according to above-mentioned 6, characterized in that the biocompatible, electrostatic aggregation-inhibiting substance having a melting point and/or phase transition temperature of 40°C or higher is one or two or more selected from the group consisting of hydrogenated lecithin, distearoyl phosphatidylcholine, cholesterol, cholesterol palmitate, cholesterol stearate, polyoxyethylene-polyoxypropylene glycol, polyethylene glycol 4000, polyethylene glycol 6000, polyethylene glycol 20000, and L-cystine,

8. a method of manufacturing a dry powder inhalation for pulmonary delivery according to above-mentioned 7, wherein the biocompatible, electrostatic aggregation-inhibiting substance having a melting point and/or phase transition temperature of 40°C or higher is one or two or more selected from the group consisting of hydrogenated lecithin, cholesterol, distearoyl-phosphatidylcholine, and polyethylene glycol 4000,

9. a method of manufacturing a dry powder inhalation for pulmonary delivery according to above-mentioned 8, wherein the biocompatible, electrostatic aggregation-inhibiting substance having a melting point and/or phase transition temperature of 40°C or higher is one or two or more selected from the group consisting of hydrogenated lecithin and cholesterol,

10. a method of manufacturing a dry powder inhalation for pulmonary delivery according to above-mentioned 7, characterized in that [the powder] contains 0.05 to 95 wt% biologically active substance and 5 to 99.95 wt% biocompatible, electrostatic aggregation-inhibiting substance having a melting point and/or phase transition temperature of 40°C or higher, it is obtained by coating the biologically active substance with this [biocompatible] substance, and it has a geometric particle diameter of 0.5 to 8 μm.

The "fine" biologically active substance used in the present invention means a biologically active substance particle that has been made into fine [particles] with a geometric particle diameter of 0.5 μm to 8 μm, preferably 0.5 μm to 5 μm, further preferably 0.5 μm to 3 μm.

The "geometric particle diameter" here represents the average particle diameter of the primary particles of the powder. It can be found by, for instance, determining Feret's diameter, which is a constant-direction diameter that is obtained by three-dimensional random arrangement of particles and measurement of particle dimension in a constant direction. Feret's diameter can be obtained by determining the maximum dimension of a particle in a constant direction. In the present invention it means the value that is obtained from the average when Feret's diameter of 100 particles is determined with an image magnified 2000 X under an electron microscope (JSM-5400, JEOL, Ltd.).

"Crystal form" means that the biologically active substance of the present invention is present as crystals and it can also include the morphology where a part of this substance is present in amorphous form. "A part" means approximately a weight ratio of 0% to approximately 30%, preferably approximately 0% to approximately 15%, further preferably approximately 0% to approximately 10%, per total weight.

Moreover, "melting point" in the present invention means in particular the temperature when the substance in the solid phase maintains equilibrium with the liquid phase, and "phase transition temperature" means the temperature at which a substance changes to a different phase, for instance, the temperature at which there is transition from the solid phase to the liquid phase, or the glass transition temperature. They are entered as "melting point and/or phase transition temperature" because there are substances that have both a melting point or a phase transition temperature. For instance, phosphatidylcholine (melting point of approximately 235°C, phase transition temperature of -15 to 1-7°C) is excluded. The substance in question is excluded when its melting point and/or phase transition temperature is 40°C or lower.

The "biocompatibility" in the present invention means a substance that is present *in vivo* or a pharmaceutically acceptable substance that dissolves and decomposes *in vivo.* Examples are those entered in the Japanese Pharmacopoeia (14th edition, Hirokawa Shoten), Dictionary of Pharmaceutical Additives (Nominating Committee, Evaluation and Registration Division, Pharmaceutical Affairs Bureau, Ministry of Health and Welfare, Japan Pharmaceutical Additives Association, editors), USP, EP, and Inactive Ingredient Guide (by Drug Information Resources), and those used as fillers for inhalations and injections are particularly ideal examples.

The "electrostatic aggregation-inhibiting substance" in the present invention means, for instance, a substance that, when coated on a fine biologically active substance in crystal form, produces a powder with an electrostatic charge of between 0 and 3 x $10^{-9}$ Q as determined by the Faraday gauge method described in the test examples given later.

The "coated" in the present invention means that the fine biologically active substance is coated with electrostatic aggregation-inhibiting substance. That is, in the present invention both the embodiment where all of the biologically active substance is coated with the above-mentioned [electrostatic aggregation-inhibiting] substance and the embodiment where a part of [the biologically active substance] is coated [with the above-mentioned electrostatic aggregation-inhibiting substance] are defined as "coated." The case where biologically active substance in crystal form is dispersed and supported in the above-mentioned [electrostatic aggregation-inhibiting] substance (so-called matrix) can also be included in the "coated" of the present invention. However, it does not include the embodiment of a matrix that contains amorphous biologically active substance. The above-mentioned embodiments that are obtained differ with the particle diameter of the biologically active substance in crystal form that is used and therefore, each of the above-mentioned embodiments that comprise biologically active substance in crystal form can also be contained in the composition that is obtained.

The "pulmonary delivery performance (respirable fraction)" in the present invention means the ratio of particles capable of aerodynamic pulmonary delivery. "Aerodynamic" means the properties of particles in air. Specifically, "pulmonary delivery performance (respirable fraction)" means the ratio of particles trapped on each plate with a cut off diameter within a range of 0.43 to 5.80 μm when determined in accordance with the cascade impactor method (U.S. Pharmacopoeia, 24th edition). Moreover, it means the ratio of powder that has reached Stage 2 when determined in accordance with the twin impinger method (US Pharmacopoeia, 23rd edition). In particular, "excellent pulmonary delivery performance" in the present invention means a percentage improvement as defined below of 30% or greater.

Percentage improvement (%) =

Respirable fraction of composition of present invention-

Respirable fraction of comparative control /

Respirable fraction of comparative control x 100

The "excellent stability" in the present invention means that aggregation of particles is not seen, even with storage of the dry powder inhalation for pulmonary delivery of the present invention under specific conditions, for instance, 40°C, and the like, or there is no reduction in the respirable fraction.

The present invention will now be described in further detail.

Geometric particle diameter of the biologically active substances submitted for production of the composition of the present invention is approximately 0.5 to approximately 8 μm, preferably approximately 0.5 μm to approximately 5 μm, further preferably approximately 0.5 μm to approximately 3 μm.

Moreover, geometric particle diameter of the dry powder inhalation for pulmonary delivery of the present invention is preferably approximately 0.5 to approximately 8 μm, further preferably approximately 0.5 μm to approximately 5 μm. In addition, the optimum geometric particle diameter is 0.5 to 3 μm. There is no difference between the geometric particle diameter of the biologically active substance and the geometric particle diameter of the dry powder inhalation for pulmonary delivery because the embodiment is either one where all of the biologically active substance is coated by very thin film of electrostatic aggregation-inhibiting substance, one where only a part of the biologically active substance is covered, or an embodiment that contains both of the above-mentioned embodiments.

The composition of the present invention is an embodiment wherein fine biologically active substance in crystal form is coated with a biocompatible, electrostatic aggregation-inhibiting substance. A structure wherein fine biologically active substance in crystal form is coated with one or more biocompatible, electrostatic aggregation-inhibiting substances, a structure wherein fine biologically active substance in crystal form is coated in multilayers with multiple biocompatible, electrostatic aggregation-inhibiting substances, and a structure wherein multiple fine biologically active substances in crystal form are coated with a biocompatible, electrostatic aggregation-inhibiting substance are given as examples. Furthermore, it is also possible to submit combinations of several of the above-mentioned compositions with different structures at the appropriate ratio in order to accomplish the desired pulmonary delivery performance.

Moreover, it is also possible to submit a mixture of large particles (hereafter referred to as carrier) represented by lactose with a geometric particle diameter of 20 μm or larger, which are generally used in the field of inhalations, and biologically active substance in crystal form that has been coated with one or more biocompatible, electrostatic aggregation-inhibiting substance. The lactose microparticles Pharmatose 325M (geometric average particle diameter of approximately 60 μm) made by DMV is given as a specific example of a carrier that is generally used in the field of inhalations.

The biocompatible, electrostatic-aggregation inhibiting substance that is used in the present invention is a substance that is present *in vivo* or a pharmaceutically acceptable substance that dissolves and decomposes *in vivo*, and is a biocompatible powder that inhibits electrostatic aggregation. Lipids, fatty acids and their esters, surfactants, polyethylene glycol, amino acids, and the like are given. Phospholipids, terpenoids, fatty acid esters, polyoxyethylene-polyoxypropylene glycol, polyethylene glycol, and amino acids can be given as specific examples. These are substances with a melting point and/or phase-transition temperature of 40°C or higher. If this temperature is lower than 40°[C], the substance will be difficult to handle during manufacture and it will not facilitate stabilization over time of the dry powder inhalation for pulmonary delivery of the present invention.

Glycerophospholipids and sphyngophospholipids, or their mixtures, are preferred, and hydrogenated lecithin and distearoyl phosphatidylcholine are particularly preferred as the phospholipid. Hydrogenated soy lecithin and hydrogenated egg yolk lecithin are included among the hydrogenated lecithins.

Sterol is preferred, and cholesterol is particularly preferred as the terpenoid.

Fatty acid ester of cholesterol is preferred, and cholesterol palmitate and cholesterol stearate, or their mixtures, are given as the fatty acid ester.

Polyoxyethylene (160)-polyoxypropylene (30) glycol (brand name: Pluronic F68, Asahi Denka Kogyo K.K.) is preferred as the polyoxyethylene-polyoxypropylene glycol.

Polyethylene glycol 4000, polyethylene glycol 6000, and polyethylene glycol 20000 are preferred as the polyethylene glycol.

L-cystine is preferred as the amino acid.

It is also possible to use one or a combination of two or more of the above-mentioned biocompatible, electrostatic aggregation-inhibiting substances as needed.

A hydrophobic substances is selected as the biocompatible, electrostatic aggregation-inhibiting substances when giving the quality of sustained release performance. "Hydrophobic" here means the quality of requiring 1000 mL or more of water or the 2nd fluid for disintegration tests (Japanese Pharmacopoeia (14th edition, Hirokawa Shoten)) to dissolve 1 g of solute within 30 minutes when vigorously shook for 30 seconds every 5 minutes at 20 ± 5°C. It means "very slightly soluble" or "practically insoluble," which are terms used to indicate solubility in the General Rules of the Japanese Pharmacopoeia.

"Sustained release performance" means that when dissolution of this substance is determined by the *in vitro* dissolution test method using a small amount of dissolution fluid assumed to be the volume of body fluid inside the lungs, there is continuous dissolution for 2 hours, preferably 6 hours, further preferably 12 hours. It further preferably means that the dissolution rate 30 minutes after starting the test is 0 to 30% and the dissolution rate 120 minutes after starting the test is 0 to 50%.

Examples of hydrophobic substances are terpenoids, phospholipids, fatty acid esters, and amino acids. Specific hydrophobic substances are cholesterol, hydrogenated lecithin, fatty acid esters of cholesterol, such as cholesterol palmitate and cholesterol stearate, L-cystine, and the like. Of course, one or any combination of two or more of the above-mentioned biocompatible hydrophobic substances can be used.

Furthermore, the amount added of biocompatible electrostatic aggregation-inhibiting substance is usually selected as needed in accordance with the biologically active substance in crystal form or medical use (indications), but it is preferably 3 to 99.95 wt%, more preferably 5 to 99.95 wt%, further preferably 7.5 to 99.5 wt%, further more preferably 10 to 95 wt%, of the total composition.

There are no particular restrictions to the biologically active substance that can be used in the powder of the present invention as long as it is a biologically active substance that is useful as an inhalation drug for local use or systemic use and it can be in crystal form. Moreover, it is possible to use one or a combination of two or more biologically active substances as needed.

For instance, corticosteroid hormones, β2 adrenoreceptor agonists, anticholinergic bronchodilators, anti-allergy drugs, antihistamines, leukotriene antagonists/inhibitors, thromboxane antagonists/inhibitors, leukotriene-thromboxane antagonists/inhibitors, 5-lipoxygenase inhibitors, phosphodiesterase IV inhibitors, phospholipase A2 inhibitors, $Ca^{2+}$ release-activated $Ca^{2+}$ channel inhibitors, adenosine A2 agonists, endothelin A agonists, antiviral agents, cystic lung disease remedies, expectorants, lung surfactants, and the like, are given as useful for local use, particularly for asthma, chronic obstructive pulmonary disease (COPD), and infection.

Fluticasone, beclomethasone, triamcinolone, flunisolide, budesonide, Obetamethasone, dexamethasone, fluocinolone, refleponide, mometazone, and the like, and their salts are given as corticosteroid hormones.

Formoterol, salbutamol, terbutaline, isoproterenol, fenoterol, adrenaline, pirbutelol, salmeterol, procaterol, proxaterol [Tr's note: Translation of phonetic characters], and the like, and their salts are given as β2 adrenoreceptor agonists.

(+)-(1S,3' R)-quinuclidin-3'-yl-1-phenyl-1,2,3,4-tetrahydroisoquinoline-2-carboxylate, ipratropium, tiotropium, and the like, and their salts are given as anticholinergic bronchodilators.

Cromoglycic acid, nedocromil, and the like, and their salts are given as anti-allergy drugs.

Ketotifen, azelastine, terfenadine, and the like, and their salts are given as antihistamines.

Pranlukast, zafirlukast, montelukast, and the like, and their salts are given as leukotriene antagonists/inhibitors.

Seratrodast, ozagrel, and the like, and their salts are given as thromboxane antagonists/inhibitors.

N-[5-[3-[4-chlorphenyl]sulfonyl]propyl]-2-(1H-tetrazol-5-ylmethoxy)phenyl]-3-[[4-(1,1-dimethylethyl)-2-thiazolyl]methoxy]benzamide, and the like, and their salts are given as leukotriene-thromboxane antagonists/inhibitors.

Zileuton, and the like, and their salts are given as 5-lipoxygenase inhibitors.

3-[4-(3-chlorophenyl)-1-ethyl-7-methyl-2-oxo-1,2-dihydro-1,8-naphthyridin-3-yl] propanoic acid, roflumilast, cilomilast, and the like, and their salts are given as phosphodiesterase IV inhibitors.

4-methyl-4'-[3,5-bis(trifluoromethyl)-1H-pyrazol-1yl]-1,2,3-thiadiazole-5-carboxanilide, and the like, and their salts are given as $Ca^{2+}$ release-activated $Ca^{2+}$ channel inhibitors.

N-[6-methoxy-5-(2-methoxyphenoxy)-2-(pyrimidin-2-yl)pyrimidin4-yl]-2-phenylethenesulfonamidate, and the like, and their salts are given as endothelin A antagonists.

Zanamivir, oseltamivir, and the like, and their salts are given as antiviral drugs.

Recombinant human deoxyribonuclease I (rhDNAase I), and the like, are given as cystic lung disease remedies.

Ambroxol, and the like, and their salts are given as expectorants.

Natural (extract) and synthetic lung surfactants, and the like, are given as lung surfactants.

Moreover, systemic use appears to be useful against a variety of illnesses, and diabetes drugs (insulin and its derivatives, and the like), analgesics (morphine, acetaminophen, and the like), anti-Parkinson's drugs (levodopa, and the like), anti-arthritis drugs (celecoxib, valdecoxib, and the like), anti-fungals (amphotericin B, faropenem sodium), pulmonary hypertension drugs (prostaglandin E1, prostaglandin 12 (velaprost sodium, and the like), and their derivatives, and the like), chemotherapeutics (interferon, cysplatin, doxorubicin, methotrexate, daunorubicin hydrochloride, fluorouracil, and the like), immunosuppresants (cyclosporin, taclorims, and the like), antitussives (codeine, dihydrocodeine, ephedrine, methyl ephedrine, and the like), vaccines (pneumococcal vaccine, and the like), and the like, are given.

Furthermore, these also include peptides and proteins (insulin, LHRH, glucagon, human growth hormone, and the like), cytokines (interferon, interleukin, and the like), genetic drugs (plasmid DNA, and the like), vectors (virus vectors, antivirus vectors, liposomes), antisenses (adenosine A1 receptor antisense), and the like

Furthermore, the biologically active substance component can also be a mixture of biologically active substances.

The following compounds A, B, C, D and E are given as preferred compounds: Compound A is N-[5-[3-[(4-chlorophenyl)sulfonyl]propyl]-2-(1H-tetrazol-5-ylmethoxy)phenyl]-3-[[4-(1,1-dimethylethyl)-2-thiazolyl]methoxy]benzamide, compound B is 4-methyl-4'-[3,5-bis(trifluoromethyl)-1H-pyrazol-1-yl]-1,2,3-thiadiazole-5-carboxanilide, compound C is 3-[4-(3-chlorophenyl)-1-ethyl-7-methyl-2-oxo-1,2-dihydro-1,8-naphthyridin-3-yl]propanoic acid, compound D is (+)-(1S,3'R)-quinuclidin-3'-yl 1-phenyl-1,2,3,4-tetrahydroisoquinoline-2-carboxylate monosuccinate, and compound E is potassium (E)-N-[6-methoxy-5-(2-methoxyphenoxy)-2-(pyrimidine-2-yl) pyrimidine-4-yl]-2-phenylethenesulfonamidate.

The amount of biologically active substance in crystal form that is added is usually the therapeutic effective amount or prophylactic effective amount selected as needed in accordance with the biologically active substance or medical use (indication). There are no set standards, but, for instance, 0.05 to 99.95 wt%, preferably 0.05 to 99.5 wt%, further preferably 0.05 to 99 wt%, particularly 0.05 to 95 wt%, of the total composition can be selected.

There are no particular limits to the method of inhalation administration of the composition of the present invention. As with ordinary inhalations, the composition of the present invention can be filled in an appropriate capsule or blister and inhaled with an appropriate inhalation device. The composition of the present invention can also be dispersed in an appropriate solvent and inhaled through a nebulizer. Moreover, it can be dispersed in a propellant gas, such as chlorine-free hydrofluorocarbon, etc., capable of liquefaction under pressurization and inhaled as a popular MDI (meter dose inhaler).

A powder capable of pulmonary delivery after dispersion using an inhalation device, etc., should be selected as the pharmaceutical preparation form of the composition of the present invention. That is, it should take on the state of a pharmaceutical preparation at the time of administration that becomes microparticles that can be present as a powder or primary particles by any means. For instance, the powder of the present invention can take on the state of a granulated product popular in the field of solid agents in order to further improve fluidity of a powder that is to be filled in a capsule. Moreover, it can also take on the state wherein an appropriate amount of the composition of the present invention is mixed with a carrier.

Conventional methods of solid formulation can be used for formulation of the composition of the present invention, and it can be used in combination with one and/or two or more additives used in the past as needed, as long as it is within a range that has no effect on intrapulmonary delivery properties of this [composition]. Binders, extenders, fillers, lubricants, flavorings, fragrances, etc., can be given as this type of additive. Specifically, lactose, mannitol, fructose, glucose, fumaric acid, starch, and gelatin are given as examples of binders, and lactose, maltose, mannitol, xylitol, glycine, aspartic acid, starch, gelatin, dextran, and citric acid are given as examples of fillers. Lactose, starch, gelatin, fumaric acid, and phosphoric acid can be given as specific examples of lubricants, and lactose, maltose, mannitol, fructose, xylitol, and citric acid can be given as specific examples of flavorings.

"Within a range that has no effect on pulmonary delivery performance" means that pulmonary delivery performance of the initial composition is not compromised.

Furthermore, the binder that can be used in the present invention is not used for the purpose of adhering the electrostatic adhesion-inhibiting agent of the present invention to the biologically active substance.

The composition of the present invention can be prepared by a simple manufacturing method. For instance, a composition can be made by suspending a biologically active substance in crystal form, which has been brought to a geometric particle diameter of 5 μm or smaller, in an appropriate solvent in which has been dissolved a biocompatible, electrostatic aggregation-inhibiting substance, such as cholesterol, and the like, and spray drying this suspension with a spray dryer and the like in order to remove the solvent. A conventional method can be used as the method of manufacturing particles with a geometric particle diameter of 5 μm or smaller. The method of manufacturing microparticles by micropulverization with a jet mill or microfluidizer and the like, a spray dryer, or means that employ a supercritical fluid such as carbon dioxide (supercritical fluid method) can be used.

An organic solvent such as ethanol or methanol, water, a supercritical fluid such as $CO_2$, and the like can be given as appropriate solvents that dissolve the biocompatible, electrostatic aggregation-inhibiting substance. Of these solvents, one that will not dissolve the biocompatible substance but will dissolve the electrostatic aggregation-inhibiting substance is selected in accordance with the biologically active substance. Moreover, even if the solvent is one that will dissolve both the biologically active substance and electrostatic aggregation-inhibiting substance, it is also possible to dissolve the electrostatic aggregation-inhibiting substance only by mixing different solvents, mixing a supercritical fluid and solvent, or adjusting the conditions, and the like. It should not be interpreted that the manufacturing method of the present invention is limited to these manufacturing methods.

Next, the method of manufacturing the dry powder inhalation for pulmonary delivery of the present invention will be described.

The biologically active substance in crystal form is pulverized under a pulverization air pressure of 5.0 b and a feed air pressure of 5.5 b with a jet mill pulverization device (Spiral Jet Mill 50AS of Hosokawa Micron Corp.) to prepare microparticles with a geometric particle diameter of 5 μm or smaller. Next, after dissolving, for instance, cholesterol (The Liposome Co., Inc.) in a mixture of ethanol and purified water, this jet mill-pulverized microparticles are added and ultrasound treated for five minutes to prepare a suspension. This suspension is spray dried under suitable conditions with, for instance, a spray dryer (for instance, DL-41 of Yamato Scientific Co., Ltd.) to obtain the dry powder inhalation for pulmonary delivery of the present invention.

Whether or not the biologically active substance is in crystal form can be confirmed using a method such as X-ray analysis or differential scanning calorimetry (DSC).

The present invention provides marked results in that it is possible to present by a simple method a dry powder inhalation for pulmonary delivery that is made from a biologically active substance in crystal form and a biocompatible, electrostatic aggregation-inhibiting substance and that has excellent safety, stability, and pulmonary delivery performance.

Moreover, it is also possible to provide sustained release performance that is appropriate for the properties of the biologically active substance by selecting [the appropriate] hydrophobic substance.

**Brief Description of the Drawings**

Figure 1 is the state before and after storage of particles of the dry powder inhalation for pulmonary delivery made in Example 4 as observed under a scanning electron microscope (SEM).
Figure 2 is the state before and after storage of particles of the dry powder inhalation for pulmonary delivery made in Comparative Example 3 as observed under a scanning electron microscope (SEM).
Figure 3 is the powder X-ray diffraction results of the powder for inhalation that was produced with a jet mill (Comparative Example 1).
Figure 4 is the powder X-ray diffraction results of the dry powder inhalation for pulmonary delivery wherein compound A jet mill-pulverized composition was coated with cholesterol (Example 1).
Figure 5 is the powder x-ray diffraction results of the powder for inhalation produced by dissolution of compound A and cholesterol together (Comparative Example 5).
Figure 6 is the state before and after storage of the particles of the dry powder inhalation for pulmonary delivery produced in Example 1 as observed under a scanning electron microscope (SEM).
Figure 7 is the state before and after storage of the particles of the dry powder inhalation for pulmonary delivery produced in Comparative Example 5 as observed under a scanning electron microscope (SEM).
Figure 8 is a graph showing the results of dissolution tests of Example 1 and Comparative Example 1.

**Preferred Embodiments of the Present Invention**

The details of the present invention are described with examples below, but it is not to be interpreted that the present invention is limited to these [examples].

Example 1. Preparation of dry powder inhalation for pulmonary delivery of Compound A (free form) micropowder coated with 25 wt% cholesterol

First, 2.4 g Compound A (free form) jet mill-pulverized product produced in Comparative Example 1 were added to a mixture of 0.6 g cholesterol (The Liposome Co., Inc.), 400 g ethanol, and 197 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air of 3 kgf/cm$^2$, drying air of 0.8 m$^3$/min, and inlet temperature of 70°C to obtain the dry powder inhalation for pulmonary delivery of the present invention.

Example 2. Preparation of dry powder inhalation for pulmonary delivery of Compound A (free form) micropowder coated with 11.5 wt% cholesterol

First, 2.7 g Compound A (free form) jet mill-pulverized product produced in Comparative Example 1 were added to a mixture of 0.3 g cholesterol (The Liposome Co., Inc.), 200 g ethanol, and 100 g purified water and ultrasound treated for five minutes to prepare a suspension with a solid concentration of 1.0 w/w%. This suspension was spray dried with a spray dryer (DL-41 of Yamato Scientific Co., Ltd.) at a spraying liquid feed rate of 2 g/min, atomizing air of 3 kgf/cm$^2$, drying air of 0.8 m$^3$/min, and inlet temperature of 67°C to obtain the dry powder inhalation for pulmonary delivery of the present invention.

Example 3. Preparation of dry powder inhalation for pulmonary delivery of Compound A (free form) micropowder coated with 5.3 wt% cholesterol

First, 2.85 g Compound A (free form) jet mill-pulverized produced in Comparative Example 1 were added to a mixture of 0.15 g cholesterol (The Liposome Co., Inc.), 200 g ethanol, and 100 g purified water and ultrasound treated for five minutes to prepare a suspension with a solid concentration of 1.0 w/w%. This suspension was spray dried with a spray dryer (DL-41 of Yamato Scientific Co., Ltd.) at a spraying liquid feed rate of 2 g/min, atomizing air of 3 kgf/cm$^2$, drying air of 0.8 m$^3$/min, and inlet temperature of 65°C to obtain the dry powder inhalation for pulmonary delivery of the present invention.

Example 4. Preparation of dry powder inhalation for pulmonary delivery of Compound A (free form) micropowder coated with 25 wt% hydrogenated lecithin

First, 0.6 g hydrogenated soy lecithin (The Liposome Co., Inc.) were dissolved in 400 g ethanol. After mixing 197 g purified water, it was heated to approximately 50°C. Then 2.4 g Compound A (free form) jet mill-pulverized product produced in Comparative Example 1 were added and ultrasound treated for five minutes while heating to approximately 50°C to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air of 3 kgf/$cm^2$, drying air of 0.8 $m^3$/min, and inlet temperature of 70°C to obtain the dry powder inhalation for pulmonary delivery of the present invention.

Example 5. Preparation of dry powder inhalation for pulmonary delivery of Compound A (free form) micropowder coated with 25 [wt]% polyethylene glycol 4000

First, 2.4 g Compound A (free form) jet mill-pulverized product produced in Comparative Example 1 were added to a mixture of 0.6 g polyethylene glycol 4000 (Kanto Kagaku), 200 g ethanol, and 397 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air of 3 kgf/$cm^2$, drying air of 0.8 $m^3$/min, and inlet temperature of 70°C to obtain the dry powder inhalation for pulmonary delivery of the present invention.

Example 6. Preparation of dry powder inhalation for pulmonary delivery of Compound A (free form) micropowder coated with 25 [wt]% pluronic F68

First, 1.6 g Compound A (free form) jet mill-pulverized product produced in Comparative Example 1 were added to a mixture of 0.4 g pluronic F68 (Asahi Denka Kogyo K.K.), 267 g ethanol, and 131 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air of 3 kgf/$cm^2$, drying air of 0.8 $m^3$/min, and inlet temperature of 70°C to obtain the dry powder inhalation for pulmonary delivery of the present invention.

Example 7. Preparation of dry powder inhalation for pulmonary delivery of Compound A (free form) micropowder coated with 11.5 wt% L-cystine

First, 0.2 g L-cystine (Nihon Rikagaku) was dissolved in 250 g 0.01-N-NaOH solution and 135 g ethanol were added and mixed. Approximately 15 ml 0.1 N-HCl were added for neutralization to weak alkalinity. Then 1.8 g Compound A (free form) jet mill-pulverized product produced were added and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air of 3 kgf/$cm^2$, drying air of 0.8 $m^3$/min, and inlet temperature of 70°C to obtain the dry powder inhalation for pulmonary delivery of the present invention.

Example 8. Preparation of dry powder inhalation for pulmonary delivery of Compound A (free form) micropowder coated with 25 wt% distearoylphosphatidylcholine

First, 2.4 g Compound A (free form) jet mill-pulverized product produced in Comparative Example 1 were added to a mixture of 0.6 g distearoylphosphatidylcholine (The Liposome Co., Inc.), 420 g ethanol, and 197 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 8 g/min, atomizing air of 3 kgf/$cm^2$, drying air of 0.8 $m^3$/min, and inlet temperature of 80°C to obtain the dry powder inhalation for pulmonary delivery of the present invention.

Example 9. Preparation of dry powder inhalation for pulmonary delivery of Compound B micropowder coated with 25 [wt]% polyethylene glycol 4000

First, 2.4 g Compound B jet mill-pulverized product produced in Comparative Example 2 were added to a mixture of 0.6 g polyethylene glycol 4000 (Kanto Kagaku), 200 g ethanol, and 397 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4 g/min, atomizing air

of 3 kgf/cm$^2$, drying air of 0.8 m$^3$/min, and inlet temperature of 70°C to obtain the dry powder inhalation for pulmonary delivery of the present invention.

Example 10. Preparation of dry powder inhalation for pulmonary delivery produced by mixing composition of Compound A (free form) micropowder coated with 25 wt% hydrogenated lecithin (Example 4) with lactose carrier

Fifty milligrams of the composition produced in Example 4 and 200 mg lactose microparticles for inhalation Pharmatose 325M (DMV) were mixed for five minutes in a co-stoppered glass centrifugation tube to obtain the dry powder inhalation for pulmonary delivery [of the present invention].

Example 11

First, 0.4 g of hydrogenated soy lecithin (The Liposome Co., Inc.), 0.2 g of cholesterol (The Liposome Co., Inc.), 400 g of ethanol, and 197 g of purified water were mixed and heated to approximately 50°C. Then 2.4 g of compound A (free form) jet mill-pulverized product (geometric particle diameter of 2.2 µm) were added and ultrasound treated for five minutes while heating to approximately 50°C to prepare a suspension with a solid concentration of 0.5 w/w%. This suspension at approximately 50°C was spray dried with a spray dryer (DL-41 of Yamato Scientific Co., Ltd.) at a spraying liquid feed rate of 8 g/min, atomizing air of 3 kgf/cm$^2$, drying air of 0.8 m$^3$/min, and inlet temperature of 80°C to obtain the sustained-release dry powder inhalation for pulmonary delivery of the present invention.

Example 12. Preparation of dry powder inhalation for pulmonary delivery of compound C fine powder coated with 25 [wt]% polyethylene glycol 4000

First, 2.4 g of compound C jet mill-pulverized product made in Comparative Example 6 were added to a mixture of 0.6 g of polyethylene glycol 4000 (Kanto Kagaku), 100 g of ethanol, and 497 g of purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 8 g/min, atomizing air of 3 kgf/cm$^2$, drying air of 0.7 m$^3$/min, and inlet temperature of 85°C to obtain the dry powder inhalation for pulmonary delivery of the present invention.

Comparative Example 1. Preparation of powder for inhalation obtained by jet mill pulverization of Compound A (free form)

Eighty grams Compound A (free form) were pulverized with a jet mill pulverization device (Spiral Jet Mill 50AS of Hosokawa Micron Corp.) at a pulverization air pressure of 5.0 b and feed air pressure of 5.5 b to obtain a powder for inhalation.

Comparative Example 2. Preparation of powder for inhalation obtained by jet mill pulverization of Compound B

Eighty grams Compound B were pulverized with a jet mill pulverization device (Spiral Jet Mill 50AS of Hosokawa Micron Corp.) at a pulverization air pressure of 5.0 b and feed air pressure of 5.5 b to obtain a powder for inhalation.

Comparative Example 3. Preparation of powder for inhalation of Compound A (free form) micropowder coated with 5 [wt]% egg yolk lecithin with a phase transition temperature lower than 40°C

First, 2.85 g Compound A (free form) jet mill-pulverized product (particle diameter of 2.7 µm) were added to a mixture of 0.15 g egg yolk lecithin (The Liposome Co., Inc.), 400 g ethanol, and 197 g purified water and ultrasound treated for five minutes to prepare a suspension with a solid concentration of 0.5 w/w%. This suspension was spray dried with a spray dryer (DL-41 of Yamato Scientific Co., Ltd.) at a spraying liquid feed rate of 4.5 g/min, atomizing air of 3 kgf/cm$^2$, drying air of 0.8 m$^3$/min, and inlet temperature of 60°C to obtain a powder for inhalation.

Comparative Example 4. Preparation of powder for inhalation by mixing Compound A (free form) jet mill-pulverized product with lactose carrier

Fifty milligrams of Compound A (free form) micropowder produced in Comparative Example 1 and 200 mg lactose microparticles (Pharmatose 325M of DMV) were mixed for five minutes in a co-stoppered glass centrifugation tube to obtain a dry powder for inhalation.

Comparative Example 5. Preparation of matrix-like powder for inhalation containing amorphous compound A made from compound A (free form)/cholesterol (8/2)

First, 2.4 g of compound A (free form), 0.6 g of cholesterol (The Liposome Co., Inc.), 507 g of ethanol, and 90 g of purified water were mixed to prepare a solution with a solid concentration of 0.5 wt%. This solution was spray dried with a spray dryer (DL-41 of Yamato Scientific Co., Ltd.) at a spraying liquid feed rate of 6 g/min, atomizing air of 1.5 kgf/cm$^2$, drying air of 0.8 m$^3$/min, and inlet temperature of 70°C to obtain a powder for inhalation.

Comparative Example 6. Preparation of powder for inhalation by jet mill pulverization of compound C

Eighty grams of compound C were pulverized at a pulverization air pressure of 4.5 b and feed air pressure of 6.2 b using a jet mill pulverization device (Spiral Jet Mill 50AS of Hosokawa Micron Corp.) to obtain a powder for inhalation.

Comparative Example 7.

First, 2.4 g of compound A (free form) jet mill-pulverized product (geometric particle diameter of 2.2 μm) were added to a mixture of 0.6 g of dipalmitoyl phosphatidylcholine (DPPC) (Nippon Fine Chemical Co., Ltd.), 400 g of ethanol, and 197 g of purified water and heated and ultrasound treated for five minute to obtain a suspension with a solid concentration of 0.5 w/w%. This solution was spray dried with a spray dryer (DL-41 of Yamato Scientific Co., Ltd.) at a spraying liquid feed rate of 4 g/min, atomizing air of 3 kgf/cm$^2$, drying air of 0.8 m$^3$/min, and inlet temperature of 65°C to obtain a powder for inhalation.

Comparative Example 8

First, 10.0 g of compound A (free form), 495 g of methanol, and 495 g of dichloromethane were mixed to prepare a solution with a solid concentration of 1.0 w/w%. This solution was spray dried with a spray dryer (DL-41 of Yamato Scientific Co., Ltd.) at a spraying liquid feed rate of 10 g/min, atomizing air of 1.0 kgf/cm$^2$, drying air of 0.8 m$^3$/min, and inlet temperature of 70°C to obtain a powder for inhalation.

Test 1. Observation of dry powder inhalations for pulmonary delivery and powders for inhalation under scanning electron microscope

The dry powder inhalations for pulmonary delivery of Examples 1 through 5 and 7, 8, 9 and 12 and powders for inhalation that were prepared in Comparative Examples 1 through 3 and 5 and 6 were observed under a scanning electron microscope (JSM-5400 of JEOL, Ltd.). Geometric particle diameter of all of the compositions and powders was 0.5 to 5 μm, and was a particle diameter appropriate for powders for inhalation.

Table 1. Geometric particle diameter of dry powder inhalations for pulmonary delivery and powders for inhalation

Table 1

| Test composition | Geometric particle diameter (μm) |
|---|---|
| Example 1 (Cholesterol 25% coating) | 2.81 |
| Example 2 (Cholesterol 11.5% coating) | 2.44 |
| Example 3 (Cholesterol 5.3% coating) | 2.22 |
| Example 4 (Hydrogenated lecithin 25% coating) | 3.76 |
| Example 5 (PEG4000 25% coating) | 2.01 |
| Example 7 (L-cystine 11.5% coating) | 1.91 |
| Example 8 (DSPC 25% coating) | 2.03 |
| Example 9 (PEG4000 25% coating) | 1.91 |
| Example 12 (PEG4000 25% coating) | 1.98 |

Table 1   (continued)

| Test composition | Geometric particle diameter (μm) |
|---|---|
| Comparative Example 1 (No coating) | 2.16 |
| Comparative Example 2 (No coating) | 0.85 |
| Comparative Example 3 (Egg yolk lecithin 6% coating) | 2.31 |
| Comparative Example 5 (Cholesterol matrix) | 2.27 |
| Comparative Example 6 (No coating) | 0.96 |

Test 2. Electrostatic charge and performance of pulmonary delivery of dry powder inhalations for pulmonary delivery and powders for inhalation

The electrostatic charge of dry powder inhalations for pulmonary delivery prepared in Examples 1, 3, 4, 5, 7, 8, 9 and 12 and the powders for inhalation prepared in Comparative Examples 1, 2 and 6 was determined by the Faraday gauge method. The electrostatic charge of 1 g after vigorously shaking and the compositions and powders in an IWAKI polypropylene centrifugation tube (Asahi Technoglass) was determined with an electrostatic charge meter (KQ-431B of Kasuga Electric Works Ltd.).

Furthermore, the respirable fraction was determined in accordance with the cascade impactor method (U.S. Pharmacopoeia, 24th edition). An appropriate amount of powder for inhalation was filled in an HPMC No. 2 capsule and charged in an inhalation device (Jethaler™ of Unisia Jecs Co., Ltd.). Weight of the particles trapped on plates with cut-off diameters of 0.43 to 5.80 μm when the device was attached to the cascade impactor and air was drawn (28.3 ml/min, 10 sec) was determined. Total particle weight on each plate with the above-mentioned cut-off diameters to the amount filled in the capsule is served as the respirable fraction.

Table 2. Results of electrostatic charge and respirable fraction of dry powder inhalations for pulmonary delivery and powders for inhalation

Table 2

| Test composition | Electrostatic charge (*10$^{-9}$Q) | Respirable fraction (%) | Percentage improvement (%) |
|---|---|---|---|
| Example 1 (Cholesterol 25% coating) | 0 | 29 | 81 |
| Example 3 (Cholesterol 5.3% coating) | - | 29 | 81 |
| Example 4 (Hydrogenated lecithin 25% coating) | 1.5 | 27 | 69 |
| Example 5 (PEG4000 25% coating) | 0.5 | 28 | 75 |
| Example 7 (L-cystine 25% coating) | 0.5 | 37 | 131 |
| Example 8 (DSPC 25% coating) | 0.5 | 34 | 113 |
| Example 9 (PEG4000 25% coating) | 0 | 25 | 317 |
| Example 12 (PEG4000 25% coating) | 0.7 | 29 | 314 |
| Comparative Example 1 (No coating) | 7.5 | 16 | - |

Table 2   (continued)

| Test composition | Electrostatic charge ($*10^{-9}Q$) | Respirable fraction (%) | Percentage improvement (%) |
|---|---|---|---|
| Comparative Example 2 (No coating) | 5.4 | 6 | - |
| Comparative Example 6 (No coating) | 3.4 | 7 | - |

The electrostatic charge of the dry powder inhalations for pulmonary delivery coated with an electrostatic aggregation-inhibiting substance was markedly reduced and their pulmonary delivery performance was significantly improved when compared to the uncoated powders for inhalation of the comparative examples (Table 2). Moreover, a good respirable fraction of 20% or higher was seen with an electrostatic charge of between 0 and $3 \times 10^{-9}Q$.

Test 3. Performance of pulmonary delivery of dry powder inhalation for pulmonary delivery produced using lactose carrier

Respirable fraction of the dry powder inhalation for pulmonary delivery of Example 10 and the powder for inhalation prepared in Comparative Example 4 was determined by the twin impinger method (U.S. Pharmacopoeia, 23rd edition). An appropriate amount of the dry powder inhalation for pulmonary delivery was packed in an HPMC No. 2 capsule and charged in an inhalation device (Jethaler™ of Unisia Jecs Co., Ltd.). The amount of Compound A delivered to Stage 2 when air was drawn (60 ml/min, 4 sec) from the device with the twin impinger was determined by HPLC. The amounts delivered to Stage 2 in contrast to the amount filled in the capsule served as the respirable fraction.

Table 3. Results of the respirable fraction of powders for inhalation produced by mixing with lactose carrier

Table 3

| Test composition | Respirable fraction (%) | Improvement (%) |
|---|---|---|
| Example 10 | 15 | 275 |
| Comparative Example 4 | 4 | - |

As shown in Table 3, surface-modified powders showed a high respirable fraction in comparison to biologically active substance microparticles that had not been surface-modified, even in the case of powders for inhalation that had been made using a carrier. It appears that in Comparative Example 4, there was very strong adhesive force of the biologically substance active microparticles to the carrier surface and that the respirable fraction was low because the biologically active substance particles could not be detached from the carrier surface during powder dispersion with the inhalation device. On the other hand, it appears that adhesive force to the carrier was reduced with the composition in Example 10 by surface modification of the biologically active substance microparticles, improving [particle] separation and dispersibility.

Test 4. Results of stability tests during storage under harsh conditions (Example 4 vs Comparative Example 3)

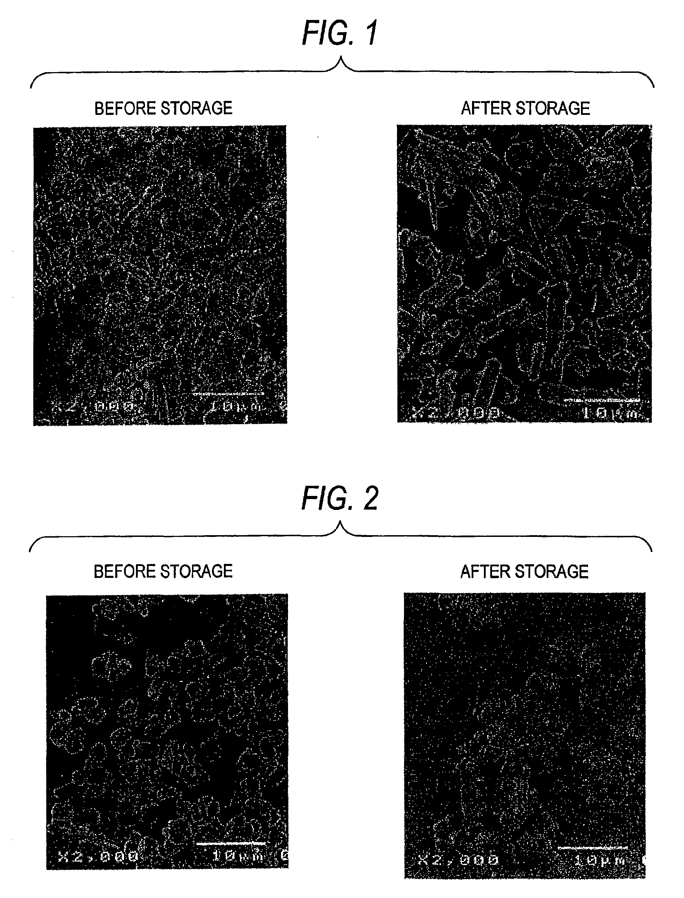

Appropriate amounts of both the dry powder inhalation for pulmonary delivery coated with hydrogenated lecithin having a phase transition temperature of 55°C (Example 4) and the powder for inhalation coated with egg yolk lecithin having a phase transition temperature of -15°C (Comparative Example 3) were filled into HPMC No. 2 capsules and the state after storage for one week at 40°C was observed under a scanning electron microscope (SEM) (JSM-5400 of JEOL, Ltd.). As shown in Figure 1, there were no changes in the microparticles before storage and after storage with the diy powder inhalation for pulmonary delivery coated with hydrogenated lecithin having a phase transition temperature of 55°C. But, as shown in Figure 2, aggregation of microparticles occurred after storage at 40°C with the powder for inhalation coated with egg yolk lecithin, which has a phase transition temperature lower than 40°C.

**EP 1 486 204 A1**

Test 5. Crystal state of dry powder inhalation for pulmonary delivery and powder for inhalation (Example 1 vs. Comparative Examples 1 and 5)

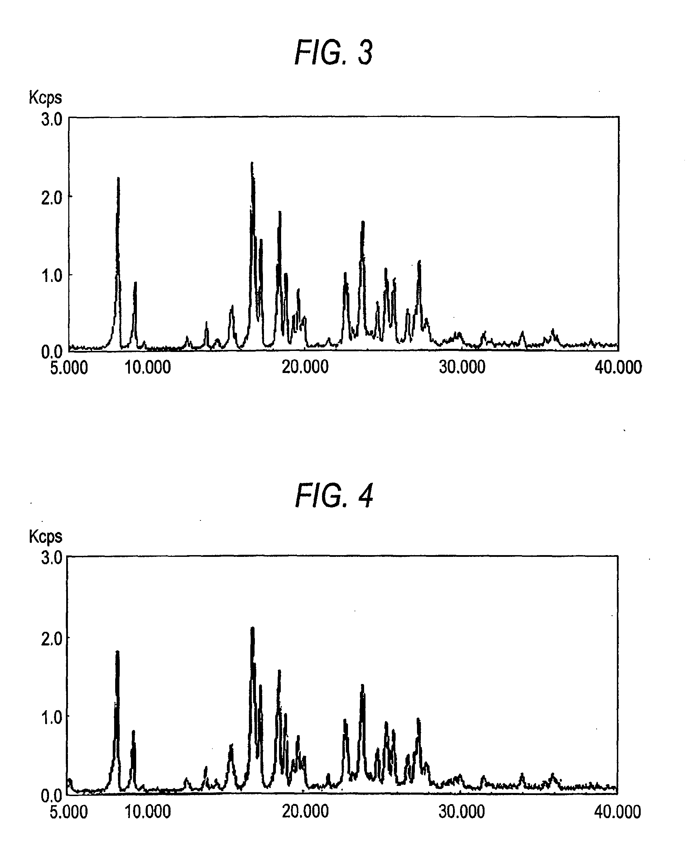

The crystal state of the dry powder inhalation for pulmonary delivery and powder for inhalation was observed by the powder X-ray diffraction method. An appropriate amount of Compound A (free form) powder for inhalation that had been made by jet mill pulverization (Comparative Example 1) was charged in a powder X-ray diffraction device (RINT-1400 of Rigaku Denki) and determined under determination conditions (tube: Cu, tube voltage: 40 kV, tube current: 40 mA, scanning speed: 3.0°/min, wavelength: 1.54056 Å). As shown in Figure 3, a peak based on the crystals of Compound A (free form) is seen, confirming a crystal state.

When the dry powder inhalation for pulmonary delivery of Compound A jet mill-pulverized composition coated with cholesterol (Example 1) was similarly tested, the same peak as in Comparative Example 1 was seen, as shown in Figure 4, confirming that there are no changes in the crystal state, even with coating with cholesterol. Furthermore, when tests were performed by a powder for inhalation consisting of Compound A and cholesterol (Comparative Example 5), a peak derived from Compound A crystals was not seen, as shown in Figure 5, and [therefore] Compound A was present in amorphous form in the composition.

Test 6. Results of stability tests during storage under harsh conditions (Example 1 vs. Comparative Example 5)

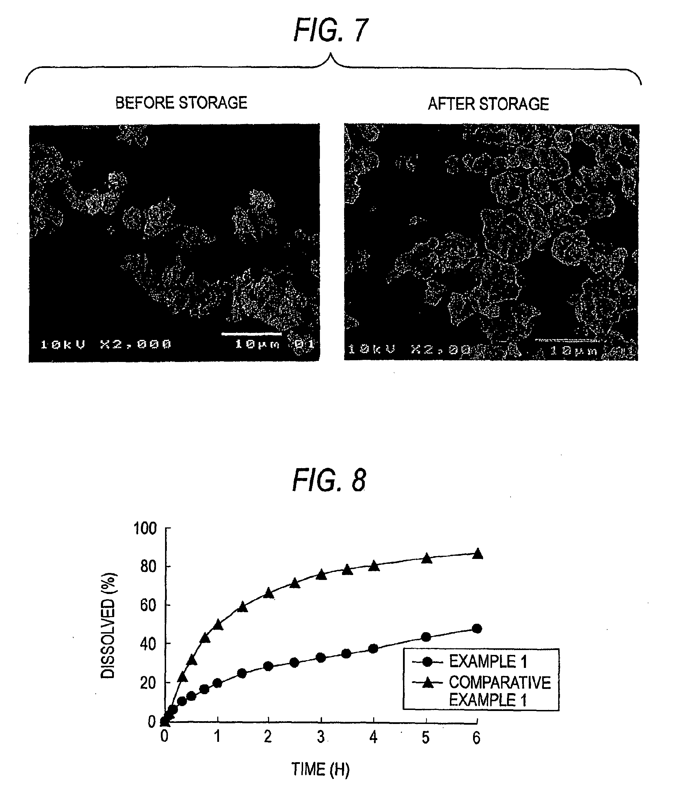

The dry powder inhalation for pulmonary delivery of crystalline Compound A coated with cholesterol (Example 1) and the powder for inhalation of amorphous Compound A dispersed in cholesterol (Comparative Example 5) were stored for seven days at 40°C and 75% [RH]. The state before and after storage was observed under a scanning electron microscope (SEM) (JSM-5400 of JEOL, Ltd.). As shown in Figure 6, there were no changes in the morphology of the microparticles before storage and after storage, and there were hardly any changes in particle diameter as well, with the dry powder inhalation for pulmonary delivery of crystalline biologically active substance coated with cholesterol substance (Example 1). On the other hand, as shown in Figure 7, aggregation between particles occurred and there was the marked increase in particle diameter after storage under harsh conditions with the powder for inhalation consisting of amorphous biologically active substance (Comparative Example 5).

Furthermore, an appropriate amount of each composition was filled in an HPMC No. 2 capsule and their respirable fraction was determined by the cascade impactor method (U.S. Pharmacopoeia, 24th edition) as in Test 2.

Table 4. Stability during storage under harsh conditions

Table 4

| Test Composition | Respirable fraction (%) | |
|---|---|---|
| | Before storage | After storage |
| Example 1 | 23 | 15 |
| Comparative Example 5 | 16 | 4 |

As shown in Table 4, even after storage under harsh conditions, the dry powder inhalation for pulmonary delivery wherein a crystalline biologically active substance has been coated with cholesterol (Example 1) showed a low reduction in its respirable fraction and good pulmonary delivery performance. On the other hand, after storage under harsh conditions, the powder for inhalation made from amorphous biologically active substance (Comparative Example 5) showed a marked reduction in its respirable fraction that reflects an increase in particle diameter and was therefore inappropriate as an inhalation.

Test 7: *In vitro* dissolution test method and results of dissolution tests of sustained-release dry powder inhalation for pulmonary delivery and powder for inhalation

Test results

The dissolution profile of compound A from the sustained-release dry powder inhalations for pulmonary delivery and powders for inhalation that were prepared in Examples 1 through 5 and 7 and Comparative Examples 1, 7, and 8 were evaluated using the Muranishi suppository release test device Desolise [*transliteration*] TDS-30P (Toyama Sangyo Co., Ltd.). The test conditions are as follows:

Dissolution test fluid: 250 ml phosphate buffer with pH of 7 containing 0.2% Tween 80
Liquid volume on donor side: 2 ml
Agitation speed: 100 rpm
Artificial membrane: Filter paper No. 1 (Advantec Toyo Kaisha, Ltd.)

Dissolution tests of the dry powder inhalations for pulmonary delivery that were prepared in Examples 1 through 5 and 7 and Comparative Examples 1, 7 and 8 were conducted. There was continuous dissolution of the dry powder inhalations for pulmonary delivery that were obtained by coating compound A fine powder in crystal form with a hydrophobic base, with the respective dissolution rate of biologically active substance 30 minutes and 120 minutes after starting dissolution test being 13% and 28% in Example 1, 9% and 29% in Example 2, 18% and 46% in Example 3, 16% and 42% in Example 4, and 20% and 39% in Example 7. On the other hand, there was fast dissolution of the uncoated composition in crystal form (Comparative Example 1, dissolution rate of biologically active substance 30 minutes and 120 minutes after starting test of 32% and 66%, respectively) and amorphous composition (Comparative Example 8, dissolution rate of biologically active substance 30 minutes and 120 minutes after starting test of 41 % and 75%, respectively). Moreover, dissolution could not be controlled sufficiently with the dry powder inhalation for pulmonary delivery coated with the hydrophilic substance polyethylene glycol 4000 (Example 5, dissolution rate of biologically active substance 30 minutes and 120 minutes after starting test of 26% and 57%, respectively). Furthermore, of the phospholipids, continuous dissolution was realized with the dry powder inhalation for pulmonary delivery coated with hydrogenated lecithin (Example 4), with the dissolution rate of biologically active substance 30 minutes and 120 minutes after starting the test being 16% and 42%, respectively, but dissolution could not be sufficiently controlled with the dry powder inhalation for pulmonary delivery coated with DPPC (Comparative Example 7), with the dissolution rate of biologically active substance 30 and 120 minutes after starting the test being 40% and 65%, respectively. These results were completely unexpected findings.

## Industrial Applicability

The present invention is useful in that it makes it possible to present by a simple method a dry powder inhalation for pulmonary delivery, which has excellent safety and stability over time in terms of pulmonary delivery performance, which is obtained by coating a biologically active substance in crystal form with a biocompatible, electrostatic aggregation-inhibiting substance, and with which the electrostatic charge of the composition as determined with a Faraday gauge is between 0 and $3 \times 10^{-9}$ Q.

## Claims

1. A dry powder inhalation for pulmonary delivery, **characterized in that** is obtained by coating a biologically active substance in crystal form having a particle diameter of 0.5 $\mu$m to 8 $\mu$m with a biocompatible, electrostatic aggregation-inhibiting substance having a melting point and/or phase transition temperature of 40°C or higher, and it has a particle diameter of 0.5 to 8 $\mu$m.

2. A dry powder inhalation for pulmonary delivery according to claim 1, wherein the biocompatible, electrostatic aggregation-inhibiting substance having a melting point and/or phase transition temperature of 40°C or higher is one or two or more selected from the group consisting of hydrogenated lecithin, distearoyl phosphatidylcholine, cholesterol, cholesterol palmitate, cholesterol stearate, polyoxyethylene-polyoxypropylene glycol, polyethylene glycol 4000, polyethylene glycol 6000, polyethylene glycol 20000, and L-cystine.

3. A dry powder inhalation for pulmonary delivery according to claim 2, wherein the biocompatible, electrostatic aggregation-inhibiting substance having a melting point and/or phase transition temperature of 40°C or higher is one or two or more selected from the group consisting of hydrogenated lecithin, cholesterol, distearoyl-phosphatidylcholine, and polyethylene glycol 4000.

4. A dry powder inhalation for pulmonary delivery according to claim 3, wherein the biocompatible, electrostatic aggregation-inhibiting substance having a melting point and/or phase transition temperature of 40°C or higher is one or two or more selected from the group consisting of hydrogenated lecithin and cholesterol.

5. A dry powder inhalation for pulmonary delivery according to claim 2, **characterized in that** it contains 0.05 to 95 wt% biologically active substance and 5 to 99.95 wt% biocompatible, electrostatic aggregation-inhibiting substance having a melting point and/or phase transition temperature of 40°C or higher, it is obtained by coating the biologically active substance with this [biocompatible]substance, and it has a geometric particle diameter of 0.5 to 8 μm.

6.  A method of manufacturing a dry powder inhalation for pulmonary delivery, **characterized in that** [the powder] contains a biologically active substance in crystal form having a particle diameter of 0.5 μm to 8 μm and/or a biocompatible, electrostatic aggregation-inhibiting substance having a melting point and/or phase transition temperature of 40°C or higher, it is obtained by coating the above-mentioned biologically active substance with this substance, and it has a geometric particle diameter of 0.5 to 8μm.

7.  A method of manufacturing a dry powder inhalation for pulmonary delivery according to claim 6, **characterized in that** the biocompatible, electrostatic aggregation-inhibiting substance having a melting point and/or phase transition temperature of 40°C or higher is one or two or more selected from the group consisting of hydrogenated lecithin, distearoyl phosphatidylcholine, cholesterol, cholesterol palmitate, cholesterol stearate, polyoxyethylene-polyoxypropylene glycol, polyethylene glycol 4000, polyethylene glycol 6000, polyethylene glycol 20000, and L-cystine.

8.  A method of manufacturing a dry powder inhalation for pulmonary delivery according to claim 7, wherein the biocompatible, electrostatic aggregation-inhibiting substance having a melting point and/or phase transition temperature of 40°C or higher is one or two or more selected from the group consisting of hydrogenated lecithin, cholesterol, distearoyl-phosphatidylcholine, and polyethylene glycol 4000.

9.  A method of manufacturing a dry powder inhalation for pulmonary delivery according to claim 8, wherein the biocompatible, electrostatic aggregation-inhibiting substance having a melting point and/or phase transition temperature of 40°C or higher is one or two or more selected from the group consisting of hydrogenated lecithin and cholesterol.

10. A method of manufacturing a dry powder inhalation for pulmonary delivery according to claim 7, **characterized in that** [the powder] contains 0.05 to 95 wt% biologically active substance and 5 to 99.95 wt% biocompatible, electrostatic aggregation-inhibiting substance having a melting point and/or phase transition temperature of 40°C or higher, it is obtained by coating the biologically active substance with this [biocompatible] substance, and it has a geometric particle diameter of 0.5 to 8 μm.

## FIG. 1

BEFORE STORAGE

AFTER STORAGE

## FIG. 2

BEFORE STORAGE

AFTER STORAGE

## FIG. 3

## FIG. 4

## FIG. 5

## FIG. 6

BEFORE STORAGE                    AFTER STORAGE

## FIG. 7

BEFORE STORAGE
AFTER STORAGE

## FIG. 8

<table>
<tr><td colspan="2" align="center">**INTERNATIONAL SEARCH REPORT**</td><td>International application No.<br>PCT/JP03/03165</td></tr>
</table>

| A. CLASSIFICATION OF SUBJECT MATTER |
|---|
| Int.Cl⁷  A61K9/72, A61K9/51, A61K47/14, A61K47/24, A61K47/28,<br>       A61K47/32 |

According to International Patent Classification (IPC) or to both national classification and IPC

| B. FIELDS SEARCHED |
|---|
| Minimum documentation searched (classification system followed by classification symbols)<br>Int.Cl⁷  A61K9/72, A61K9/51, A61K47/14, A61K47/24, A61K47/28,<br>       A61K47/32 |
| Documentation searched other than minimum documentation to the extent that such documents are included in the fields searched |
| Electronic data base consulted during the international search (name of data base and, where practicable, search terms used) |

C. DOCUMENTS CONSIDERED TO BE RELEVANT

| Category* | Citation of document, with indication, where appropriate, of the relevant passages | Relevant to claim No. |
|---|---|---|
| X | EP 1136068 A2  (JCR PHARM CO., LTD.),<br>26 September, 2001 (26.09.01),<br>Full text; particularly, Claims 27, 32; examples 3, 7<br>& US 2002/0009789 A    & US 2002/0058624 A<br>& JP 2002-187852 A | 1-10 |
| X<br>A | WO 01/13893 A1  (ADVANCED IHHALATION RES INC.),<br>01 March, 2001 (01.03.01),<br>Full text; particularly, example 3<br>& AU 2000/69296 B    & EP 1210068 A2<br>& JP 2003-507412 A | 1-3,5-8,10<br>4,9 |

☒ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>18 June, 2003 (18.06.03) | Date of mailing of the international search report<br>01 July, 2003 (01.07.03) |
|---|---|
| Name and mailing address of the ISA/<br>  Japanese Patent Office | Authorized officer |
| Facsimile No. | Telephone No. |

Form PCT/ISA/210 (second sheet) (July 1998)

## INTERNATIONAL SEARCH REPORT

| | International application No. |
|---|---|
| | PCT/JP03/03165 |

**C (Continuation).   DOCUMENTS CONSIDERED TO BE RELEVANT**

| Category* | Citation of document, with indication, where appropriate, of the relevant passages | Relevant to claim No. |
|---|---|---|
| X | WO 01/13891 A2 (ADVANCED IHHALATION RES INC.), 01 March, 2001 (01.03.01), Full text; particularly, Claims 8, 28; tables 2, 7(C, D) & AU 2000/69259 B        & US 2001/0036481 A & EP 1210067 A2        & JP 2003-507410 A | 1-10 |
| X A | WO 01/12160 A1 (MAINELAB), 22 February, 2001 (22.02.01), Full text; particularly, Claim 6; examples 2, 3 & FR 2797398 A1        & AU 2000/70104 B & EP 1204409 A1        & JP 2003-506479 A | 1,5,6,10 2-4,7-9 |
| X A | JP 11-302156 A (TANABE SEIYAKU CO.), 02 November, 1999 (02.11.99), Full text; particularly, examples 1 to 7 (Family: none) | 1,2,5-7,10 3,4,8,9 |
| X | EP 486959 A1 (VECTORPHARMA INT SPA), 27 May, 1992 (27.05.92), Full text; particularly, page 5, lines 3 to 4; example 17 & JP 4-283510 A        & US 5536508 A & US 5700486 A | 1,2,5-7,10 3,4,8,9 |
| A | WO 97/26863 A1 (BYK GULDEN LOMBERG CHEM FAB), 31 July, 1997 (31.07.97), & AU 9715930 B        & EP 877602 A1 & JP 2000-503976 A        & US 6315983 A | 1-10 |
| A | WO 01/12155 A1 (LIPOCINE INC.), 22 February, 2001 (22.02.01), & AU 2000/60838 B        & US 2001/0024658 A & US 6309663 A        & EP 1210063 A1 & US 6458383 A        & JP 2003-506476 A | 1-10 |
| A | WO 93/25198 A1 (TEIJIN LTD.), 23 December, 1993 (23.12.93), & AU 9343556 B        & EP 611567 A1 & US 5972388 A | 1-10 |
| A | WO 99/60999 A1 (EUROCELTIQUE SA), 02 December, 1999 (02.12.99), & AU 9943706 B        & EP 1079807 A1 & JP 2002-516266 A | 1-10 |
| A | WO 92/6703 A1 (LIPOSOME CO., INC.), 30 April, 1992 (30.04.92), & AU 9189291 B        & EP 553272 A1 & JP 6-502427 A | 1-10 |
| A | WO 99/16422 A1 (INHALE THERAPEUTIC SYSTEMS INC), 08 April, 1999 (08.04.99), & AU 9896772 B        & EP 1019021 A1 & US 6309623 A | 1-10 |

Form PCT/ISA/210 (continuation of second sheet) (July 1998)

## INTERNATIONAL SEARCH REPORT

| | International application No. |
|---|---|
| | PCT/JP03/03165 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 99/16421 A1   (INHALE THERAPEUTIC SYSTEMS INC),<br>08 April, 1999 (08.04.99),<br>& AU 9896770 B          & EP 1019020 A1<br>& US 6433040 A | 1-10 |
| A | WO 00/00215 A1   (INHALE THERAPEUTIC SYSTEMS INC.),<br>06 January, 2000 (06.01.00),<br>& AU 9935469 B          & EP 1091755 A1<br>& US 6565885 A | 1-10 |

Form PCT/ISA/210 (continuation of second sheet) (July 1998)

**INTERNATIONAL SEARCH REPORT**

International application No.

PCT/JP03/03165

<Subject of search>
Only part of the "biocompatible electrostatic aggregation inhibitors having a melting point and/or a phase transition temperature of 40 ° C or above" as specified in claims 1, 5, 6 and 10 are supported by the description in the meaning as described in PCT Article 6 and disclosed therein in the meaning as described in PCT Article 5.

Among the above-described "electrostatic aggregation inhibitors", therefore, the search was made mainly on the cases with the use of the compounds as specified in claims 2 to 4 and 7 to 9 such as those employed in Examples in the description in practice.

Form PCT/ISA/210 (extra sheet) (July 1998)